# EUROPÄISCHE PATENTSCHRIFT

(11) **EP 2 181 679 B1**
(45) Veröffentlichungstag und Bekanntmachung des Hinweises auf die Patenterteilung: **24.08.2011**
(21) Anmeldenummer: 09173307.1
(22) Anmeldetag: 16.10.2009
(51) Int. Cl.: A61F 6/00, A44C 5/00

(54) **Präservativhalteband**
Condom retaining strap
Bande de retenue pour préservatif

(30) Priorität: 03.11.2008 DE 102008054302
(43) Veröffentlichungstag der Anmeldung: 05.05.2010
(73) Patentinhaber: Stürzer, Martin, 85658 Egmating (DE)
(72) Erfinder: Stürzer, Martin, 85658 Egmating (DE)
(74) Vertreter: Maiwald Patentanwalts GmbH

(56) Entgegenhaltungen:
- DE-A1- 2 905 968
- DE-U1- 29 814 441
- FR-A3- 2 729 276
- GB-A- 166 194

## Beschreibung

### GEBIET DER ERFINDUNG:

Die Erfindung betrifft die Aufbewahrung und den Transport von Einwegprodukten. Insbesondere betrifft die Erfindung ein Präservativhalteband für die Aufbewahrung und den Transport eines Präservativs sowie die Verwendung eines Präservativhaltebandes zur Aufbewahrung und zum Transport eines Einwegproduktes.

### TECHNISCHER HINTERGRUND:

Einwegprodukte, die ein Benutzer bei sich tragen muss, werden heutzutage beispielsweise in einer Tasche transportiert. Wechselt der Benutzer seine Kleidung oder vergisst er seine Tasche ist es leicht möglich, dass er das Einwegprodukt darin vergisst und somit bei Bedarf nicht mehr darauf zugreifen kann. Dies kann insbesondere bei Präservativen zu Problemen führen.

### DARSTELLUNG DER ERFINDUNG:

Es kann als eine Aufgabe der Erfindung angesehen werden, eine einfache Vorrichtung zur Aufbewahrung und zum Transport eines Präservativs anzugeben.

Es ist ein Präservativhalteband für die Aufbewahrung und den Transport eines Präservativs am Handgelenk sowie die Verwendung eines solchen Präservativhaltebandes zur Aufbewahrung und zum Transport eines Einwegproduktes angegeben.

Die beschriebenen Ausführungsbeispiele betreffen gleichermaßen das Präservativhalteband, sowie die Verwendung des Präservativhaltebandes.

Es sei darauf hingewiesen, dass im Kontext der vorliegenden Erfindung die folgenden Definitionen benutzt werden.

### Durchtrennung:

Der Begriff Durchtrennung umfasst dabei sowohl einen Einschnitt am Präservativhalteband als auch eine kleine oder große flächige Ausstanzung und jede andere Ausnehmung oder Aussparung, die geeignet ist, dass ein verpacktes Präservativ durch diese Durchtrennung durchführbar ist und dort fixiert werden kann.

### Flexibel und Flexibilität:

Der Begriff flexibel charakterisiert im Kontext der Erfindung den entsprechenden Bandabschnitt derart, dass er einen biegsamen und verformbaren Abschnitt darstellt. Dabei ist das Zusammenwirken des Elastizitätsmoduls und der Festigkeit des verwendeten Materials mit der Geometrie des Bandabschnitts derart, dass im Bereich der Durchtrennung eine Öffnung beispielsweise durch eine Spreizung zur Durchführung des Präservativs erfolgen kann. Der flexible Bandabschnitt ist somit mechanisch nicht starr. Die verschiedenen Bereiche des Bandabschnitts, welche durch die Durchtrennung entstehen sind auf Grund der Flexibilität des Bandabschnitts gegeneinander beweglich.

### Elastisch und Elastizität:

Elastizität beschreibt im Kontext der Erfindung die Eigenschaft des Präservativhaltebandes, des Bandabschnitts, oder des verwendeten Materials, unter Krafteinwirkung seine Form zu verändern und bei Wegfall der einwirkenden Kraft in die Ursprungsform zurückzukehren. Es findet somit keine plastische Verformung statt.

Gemäß der Erfindung ist ein Präservativhalteband für die Aufbewahrung und den Transport eines Präservativs an einem Handgelenk angegeben welches die Merkmale von Anspruch 1 aufweist.

Die Fixierung kann beispielsweise auf Grund von Rückstellkräften in dem flexiblen Bandabschnitt erfolgen. Diese Rückstellkräfte können durch eine Elastizität des flexiblen Bandabschnitts verursacht sein.

Aufgrund der Flexibilität des Bandabschnitts, in dem sich die erste Durchtrennung befindet, kann mit menschlicher Kraft ein Ein- und Ausführen des verpackten Präservativs durch die erste Durchführung hindurch erfolgen. Es sind dabei keine zusätzlichen technischen Mechanismen zur Halterung, Fixierung oder den Transport nötig. Dabei ist das Präservativhalteband derart ausgeführt, dass keine Gefahr der mechanischen Zerstörung des Präservativs oder der Verpackung durch das Präservativhalteband besteht. Es weist somit keine zur Beschädigung geeigneten scharfen Ecken und Kanten auf. Dies kann die Gefahr der Beschädigung bei einem Transport reduzieren.

Weiterhin ist das Präservativhalteband aus einem Material gefertigt, das beispielsweise unempfindlich gegen Luftfeuchtigkeit ist und eine flüssigkeitsabweisende Eigenschaft aufweist. Somit kann das Präservativhalteband gefahrenlos und ohne Beschädigung beispielsweise im Nassbereich eines Schwimmbads benutzt werden. Dabei kann das Präservativhalteband weiterhin derart ausgeführt sein, dass es ohne weitere nötige Vorkehrungen am Handgelenk des Verwenders angebracht werden kann.

Mit anderen Worten stellt das Präservativhalteband eine vereinfachte, kostengünstig zu produzierende und leicht handhabbare Vorrichtung zur Aufbewahrung und zum Transport eines Präservativs dar. Sowohl eine schnelle Anbringung und Entfernung des Haltebandes an dem Handgelenk des Verwenders als auch die vereinfachte Anbringung des Präservativs an dem Halteband ist möglich. Zur Fixierung des Präservativs ist lediglich die Durchführung durch die Durchtrennung erforderlich, was eine schnelle und vereinfachte Handhabung und Austausch eines Präservativs ermöglicht.

Mit anderen Worten weist das Präservativhalteband eine Oberfläche mit einer äußeren Teiloberfläche und einen inneren Teiloberfläche auf und die Durchtrennung erstreckt sich von der äußeren Oberfläche durch das Präservativhalteband bis hin zur inneren Teiloberfläche. Es können sich aufgrund der ersten Durchtrennung der Oberfläche zumindest zwei Lamellen oder Stege im flexiblen Bandabschnitt ausbilden, die durch eine Aufspreizung einen Zwischenbereich umschließen. Das Präservativhalteband ist dabei derart ausgeführt, dass zu einer fixierenden Aufnahme des verpackten Präservativs lediglich ein Einführen des Präservativs in den Zwischenbereich nach einer Spreizung ausreicht. Aufgrund des flexiblen und/oder elastischen Bandabschnitts umschließen die zwei lamellenartigen zwei Bereiche in dem Bandabschnitt, die durch die Durchtrennung ausgebildet werden, das verpackte Präservativ und halten es an seiner Position. Dabei können beispielsweise Rückstellkräfte, die durch eine Elastizität des Bandabschnitts verursacht werden, ursächlich für die Fixierung sein.

Das Präservativhalteband ist zum schnellen und einfachen Anbringen an einem Handgelenk sowie zum schnellen und einfachen Entfernen von dem Handgelenk ausgeführt, ohne dass eine spezielle Öffnungsvorrichtung vorgesehen ist. Ebenso kann das Präservativ vor und auch nach der Anbringung des Haltebands am Armgelenk schnell und einfach an dem flexiblen Bandabschnitt angebracht werden und ebenso einfach wieder abgenommen werden. Insbesondere ist eine einhändige Bedienung des Haltebandes möglich.

Dabei sei weiterhin angemerkt, dass dieses und jedes andere Ausführungsbeispiel der Erfindung auch an einem Fußgelenk, an anderen Armbereichen als dem Handgelenk und beispielsweise, bei entsprechender Größenanpassung des Bandes, auch am Hals verwendet werde kann.

Mit anderen Worten ist das Präservativhalteband ein Armband für ein menschliches Handgelenk. Dabei kann das Präservativhalteband also das Armband in einer offenen Form vorliegen. Aber auch eine geschlossene Form, beispielsweise eine ringförmig geschlossene Form oder nahezu ringförmige geschlossene Form des Armbandes ist möglich.

Die Ausführung des Präservativhaltebandes als Armband für ein menschliches Handgelenk hat zur Folge, dass das Präservativhalteband in angebrachtem Zustand das Handgelenkvollständig umschließt, ohne dass es um das Handgelenk gewickelt werden muss. Mit anderen Worten ergibt sich durch die armbandförmige Ausführung des Präservativhaltebandes ein selbsttragender Sitz des Präservativhaltebandes an dem Handgelenk. Das Präservativhalteband muss nicht von dem Benutzer dauerhaft gegriffen werden, um dauerhaft am Handgelenk befestigt zu sein, sondern hält durch seine äußere Form selbständig, wenn der Benutzer seine Hand durch das Armband hindurch gesteckt hat; wie üblich bei einem Armband. Das Präservativhalteband muss also nicht verknotet oder um das Handgelenk umschlugen werden, um einen Halt daran hervorzurufen. Dies wird schon durch seine Armbandform gewährleistet. Zusätzliche Halte- oder Befestigungsmittel sind somit nicht notwendig.

Ein besonderer Vorteil des Präservativhaltebandes, das als Armband für ein menschliches Handgelenk ausgeführt ist, ist die Möglichkeit, das Präservativhalteband schnell und einfach über das Handgelenk überziehen zu können und damit am Handgelenk zu befestigen. Ebenso ist ein einfaches und schnelles Entfernen des Armbandes und damit des daran fixierten Präservativs möglich.

Gemäß einem weiteren Ausführungsbeispiel der Erfindung weist das Präservativhalteband einen Durchmesser auf, der nahezu einem Durchmesser eines menschlichen Handgelenks entspricht.

Sollte das Präservativhalteband zum Beispiel nahezu ringförmig, elliptisch oder in sonstiger geschlossener Armbandform vorliegen wird, so definiert das Präservativhalteband eine Öffnung zum Durchführen der menschlichen Hand, wobei diese Öffnung den genannten Durchmesser aufweist.

Es wird dadurch eine Vorrichtung bereitgestellt, die ein zumindest teilweises elastisches Armband darstellt, mit welchem eine grifflose Befestigung des Armbandes und damit des Präservativs am Handgelenk des Benutzers ermöglicht. Gemäß einem weiteren Ausführungsbeispiel der Erfindung weist das Präservativhalteband weiterhin eine zweite Durchtrennung in dem flexiblen Bandabschnitt auf, wobei der flexible Bandabschnitt eine Breite und eine Länge aufweist. Weiterhin sind die erste und die zweite Durchtrennung in der Breite nebeneinander angeordnet und in Längsrichtung wenigstens teilweise auf gleicher Höhe angeordnet. Dabei sind die erste und zweite Durchtrennung derart ausgeführt, dass ein verpacktes Präservativ sowohl durch die erste als auch durch die zweite Durchtrennung durchführbar ist und die erste und zweite Durchtrennung sind derart ausgeführt, dass das verpackte Präservativ nach einer Durchführung in dem flexiblen Bandabschnitt fixierbar ist.

Beispielsweise können sich aufgrund der beiden Durchtrennungen Lamellen oder auch Stege in dem flexiblen Bandabschnitt herausbilden. Diese Lamellen können aufgrund der Flexibilität oder auch der Elastizität des Bandabschnitts gegeneinander beweglich sein. Eine Fixierung auf Grund von elastischen Rückstellkräften in dem flexiblen Bereich ist möglich. Im Falle eines flexiblen Bandabschnitts, der plastische Eigenschaften aufweist kann die Fixierung mittels einer vollumfänglichen oder teilweisen Umschließung oder eines Formschlusses des Präservativs durch die Lamellen erfolgen.

Ebenso ist es möglich, dass die Oberflächenbeschaffenheit des Bandabschnitts im Vergleich zum restlichen Teil des Präservativhaltebandes erhöht ist, um eine verbesserte Fixierung aufgrund erhöhter Reibungskräfte zu ermöglichen.

Dabei können die erste und die zweite Durchtrennung verschiedene Längen aufweisen und in verschiedenen Winkeln zu den Rändern des Präservativhaltebandes aufweisen. Auch ein Versatz der beiden Durchtrennungen entlang der Längsrichtung ist möglich. Lediglich ein Überlapp zwischen den beiden Durchtrennungen muss derart gegeben sein, dass der überlappende Bereich in der Lage ist, die Durchführung und Fixierung des verpackten Präservativs mit einer vorgegebenen Breite zu ermöglichen.

Weiterhin ist es möglich, dass in den Ausführungsformen mit einer Durchtrennung und auch mit zwei Durchtrennungen verschiedene Bereiche des Präservativhaltebandes aus elastischem Material bestehen. Beispielsweise kann lediglich der flexible Bandabschnitt aus elastischem Material bestehen.

Gemäß der vorliegenden Erfindung besteht das Präservativhalteband aus einem elastischen Material.

Dabei erfolgt die Fixierung auf Grund von elastischen Rückstellkräften in dem flexiblen Bandabschnitt und/oder in den verschiedenen Bereichen des Bandabschnitts, die durch die Durchtrennung gebildet werden. Damit ist sowohl das gesamte Präservativhalteband als auch der flexible Bandabschnitt in der Lage, unter Krafteinwirkung seine Form zu verändern und bei Wegfall der einwirkenden Kraft in die Ursprungsform zurückzukehren. Eine plastische Verformung des Präservativhaltebandes findet somit zu keinem Zeitpunkt statt. Neben der vereinfachten Fixierung des Präservativs an dem Halteband ist auch eine vereinfachte Anbringung des Haltebandes am Handgelenk des Verwenders gewährleistet. Die Herstellung des Präservativhaltebandes aus einem elastischen Material kann es beispielsweise ermöglichen, eine Universalgröße herzustellen, die für eine Vielzahl unterschiedlicher Verwender und deren individuelle Handgelenksgrößen zu benutzen ist.

Gemäß einem weiteren Ausführungsbeispiel der Erfindung ist das Präservativhalteband aus einem Material gefertigt, welches ausgewählt ist aus der Gruppe bestehend aus Kautschuk, Latex, Gummiharz, andere verarbeitete Kautschuk ähnliche Pflanzensäfte, vulkanisiertes Material mit und ohne synthetischen Kautschukanteilen, und Material, das beim Eintrocknen durch Polymerisation zu einem elastischen Feststoff verhärtet ist.

Dabei kann bei der Verwendung der oben genannten Materialien beispielsweise auf eine möglichst angepasste Elastizitätseigenschaft des Präservativhaltebandes abgezielt werden.

Gemäß einem weiteren Ausführungsbeispiel der Erfindung sind die erste und die zweite Durchtrennung im Wesentlichen parallel entlang der Längsrichtung des Präservativhaltebandes verlaufend.

Dabei können auch, falls gewünscht, kleine Winkelabweichungen zwischen den Verläufen der ersten und der zweiten Durchtrennung vorhanden sein.

Gemäß einem weiteren Ausführungsbeispiel der Erfindung weisen die erste und die zweite Durchtrennung im Wesentlichen die gleiche Länge auf.

Um die Ästhetik des Präservativhaltebandes zu erhöhen, können beispielsweise die beiden Durchtrennungen die gleiche Länge aufweisen und parallel zueinander angeordnet sein. Dabei kann die Länge der Durchtrennungen beispielsweise durch die Breite des verpackten Präservativs bestimmt sein, damit eine problemfreie Einführung dessen möglich ist. Die Länge der beiden Durchtrennungen kann aber auch größer oder kleiner als die Breite des verpackten Präservativs sein.

Gemäß der Erfindung ist das Präservativhalteband einstückig ausgeführt.

Dabei können unterschiedliche Herstellungsverfahren zur einstückigen Produktion des Präservativhaltebandes benutzt werden. Beispielsweise ist eine Herstellung durch Formguß wie beispielsweise Spritzguss oder Druckguss möglich. Ebenso benötigt das Präservativhalteband durch die einstückige Ausführung keinerlei Verschlussmechanismen.

Gemäß einem weiteren Ausführungsbeispiel der Erfindung ist das Präservativhalteband ringförmig geschlossen ausgeführt.

Aufgrund der ringförmigen Form des Präservativhaltebandes ist eine erhöhte Anpassungsfähigkeit an verschiedene individuelle Handgelenke möglich. Ebenso wird eine rotationssymmetrische Positionierung des Präservativhaltebandes am Handgelenk ermöglicht.

Gemäß einem weiteren Ausführungsbeispiel der Erfindung weist das Präservativhalteband weiterhin eine weitere Durchtrennung an einer von der ersten Durchtrennung in Längsrichtung beabstandeten Stelle des Präservativhaltebandes auf.

Dabei kann es sich bei dieser Durchtrennung um jede Art von Aussparungen oder Ausnehmungen handeln. Im Falle eines ringförmig geschlossenen Haltebandes kann beispielsweise an von der ersten Durchtrennung gegenüberliegenden Seite des Bandes eine weitere Durchtrennung angeordnet sein.

Gemäß einem weiteren Ausführungsbeispiel der Erfindung ist die weitere Durchtrennung zur Aufnahme eines Rings oder eines Schlüssels ausgeführt.

Gemäß einem weiteren Ausführungsbeispiel der Erfindung bilden sich aufgrund der ersten Durchtrennung zumindest zwei Lamellen an der Oberfläche des flexiblen Bandabschnitts aus, wobei die beiden Lamellen zur Durchführung des verpackten Präservativs ausgeführt sind. Weiterhin erfolgt mittels der Durchführung eine Fixierung des verpackten Präservativs aufgrund einer Flexibilität der beiden Lamellen.

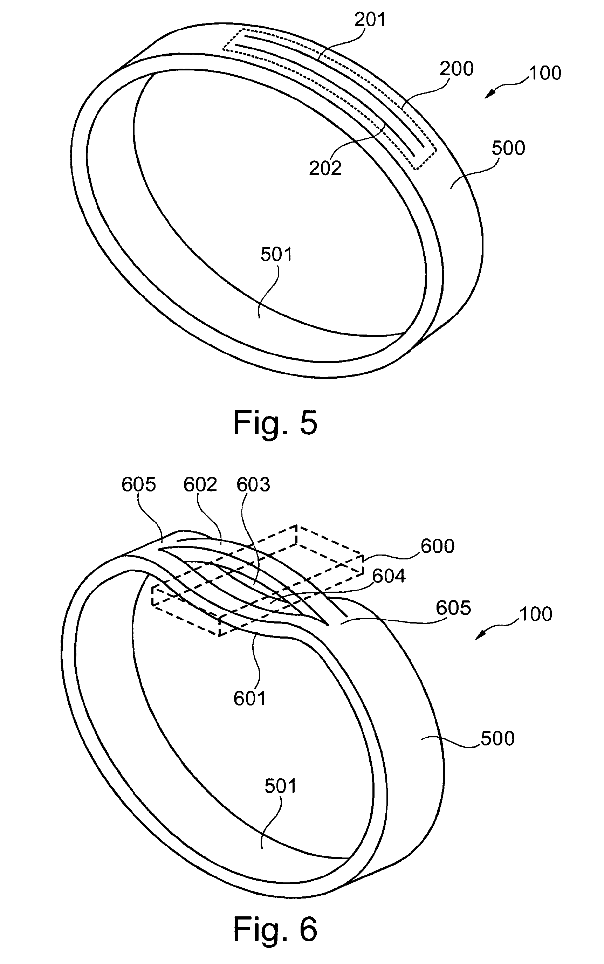

Wie beispielsweise in Fig. 6 verdeutlicht ist, ermöglichen Lamellen oder Stege die Fixierung des zu haltenden und transportierenden Kondoms. Dabei können die Kräfte, die zur Fixierung von dem Präservativhalteband auf das verpackte Präservativ wirken, in ihrem Betrag so groß sein, dass Erschütterungen während eines durchschnittlichen Transports am Handgelenk des Benutzers keinerlei Einfluss auf die Position des Präservativs haben. Rückstellkräfte in den Lamellen können dabei die Ursache für die Fixierung sein.

Gemäß einem weiteren Ausführungsbeispiel der Erfindung ist die Verwendung eines Präservativhaltebandes zur Aufbewahrung und zum Transport eines Einwegproduktes angegeben.

Dabei kann unter dem Begriff Einwegprodukt beispielsweise eine Streichholzschachtel mit Streichhölzern verstanden werden. Weiterhin kann das Präservativhalteband ebenso zur Aufbewahrung und zum Transport von beispielsweise Medikamenten, Geld, Feuerzeugen, Schlüsseln, Geld- und Kreditkarte, MP3-Playern oder Mobiltelefonen verwendet werden.

Weitere Ausführungsbeispiele und Vorteile der Erfindung ergeben sich aus der folgenden Figurenbeschreibung. Die Erfindung beschränkt sich aber nicht auf diese Ausführungsbeispiele.

Die Darstellungen in den Figuren sind schematisch und nicht maßstäblich. Bezugszeichen sind explizit nicht als einschränkend anzusehen.

### KURZE BESCHREIBUNG DER FIGUREN:

Fig. 1 bis Fig. 4 zeigen schematische zweidimensionale Darstellungen eines Präservativhaltebandes gemäß einem Ausführungsbeispiel der Erfindung.
Fig. 5 und Fig. 6 zeigen schematische, dreidimensionale Darstellungen eines Präservativhaltebandes gemäß einem Ausführungsbeispiel der Erfindung.
Fig. 7 bis Fig. 10 zeigen schematische, zweidimensionale Darstellungen eines Ausführungsbeispiels der Erfindung.
Fig. 11 bis 16 zeigen schematische, dreidimensionale Darstellungen eines Präservativhaltebandes gemäß mehreren Ausführungsbeispielen der Erfindung.

### DETAILIERTE BESCHREIBUNG DER FIGUREN:

Im Folgenden werden mit Verweis auf die Figuren bevorzugte Ausführungsbeispiele der vorliegenden Erfindung beschrieben. In der folgenden Figurenbeschreibung werden für die gleichen oder ähnlichen Elemente die gleichen Bezugsziffern verwendet.

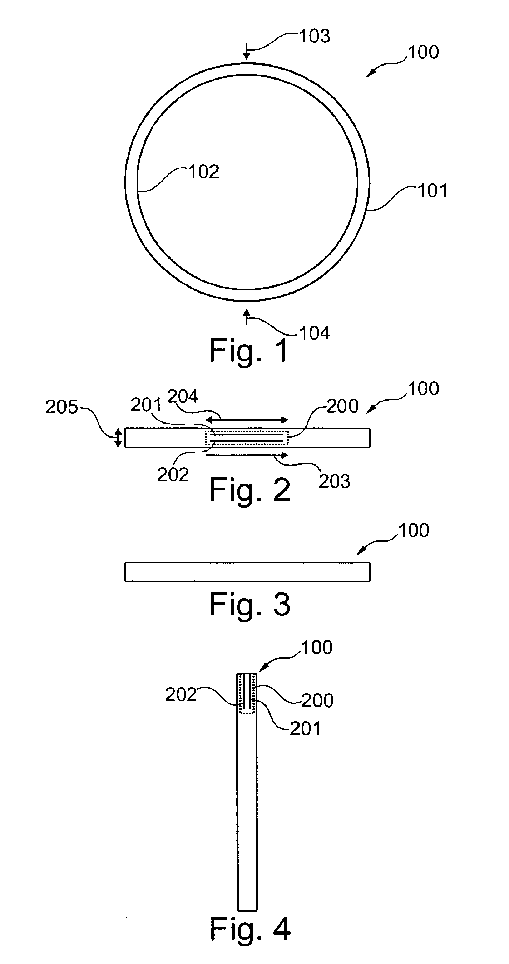

Fig. 1 zeigt eine Frontansicht eines Präservativhaltebandes 100 gemäß einem Ausführungsbeispiel der Erfindung. Es ist eine äußere Teiloberfläche, mit einer Kontur 101 und eine innere Teiloberfläche mit einer Kontur 102 gezeigt. Fig. 2 zeigt eine Draufsicht eines Präservativhaltebandes 100 der Fig. 1 in Blickrichtung 103, in welcher der flexible Bandabschnitt 200 zu sehen ist. In dem flexiblen Bandabschnitt 200 ist eine erste Durchtrennung 201 und eine zweite Durchtrennung 202 gezeigt. Diese erstrecken sich entlang der Längsrichtung 203 des Präservativhaltebandes. Der flexible Bandabschnitt 200 weist eine Länge 204 und eine Breite 205 auf. In diesem Ausführungsbeispiel ist deutlich zu sehen, dass die beiden Durchtrennungen, die hier als Schnitte ausgeführt sind, beispielsweise aber auch Ausstanzungen sein können, parallel angeordnet sind und die gleiche Länge haben.

Ein besonderer Vorteil des Präservativhaltebandes 100, das als Armband für ein menschliches Handgelenk ausgeführt ist, ist die Möglichkeit, das Präservativhalteband schnell und einfach über das Handgelenk überziehen zu können und damit am Handgelenk zu befestigen. Ebenso ist ein einfaches und schnelles Entfernen des Armbandes und damit des daran fixierten Präservativs möglich.

Gemäß dem hier gezeigten Ausführungsbeispiel der Erfindung weist das Präservativhalteband 100 einen Durchmesser auf, der nahezu einem Durchmesser eines menschlichen Handgelenks entspricht. Dadurch wird die Durchführung des Handgelenks durch die gezeigt große mittige Öffnung des Armbandes 100 ermöglicht und eine grifflose Fixierung des Armbandes an dem menschlichen Handgelenk kann erfolgen, ohne dass weiter Befestigungsmittel oder Vorrichtungen dazu verwendet werden müssen.

Fig. 3 zeigt eine Ansicht des Präservativhaltebandes 100 der Fig.1 in Blickrichtung 104. In diesem gezeigten Ausführungsbeispiel weist das Band lediglich zwei Durchtrennungen im Bereich des flexiblen Bandabschnitts auf, der hier gezeigte untere Bodenbereich sind keine weiteren Durchtrennungen angeordnet.

Fig. 4 zeigt eine Seitenansicht des Präservativhaltebandes 100 der Fig.1, wobei der flexible Bandabschnitt 200 mit der ersten und der zweiten Durchtrennung 201 und 202 zu erkennen sind.

Fig. 5 zeigt eine schematische, dreidimensionale perspektivische Ansicht des Präservativhaltebandes 100 der Fig.1, welches einen elastischen Bandabschnitt 200 mit einer ersten Durchtrennung 201 und einer zweiten Durchtrennung 202 aufweist. Durch die perspektivische Ansicht sind die äußere Teiloberfläche 500 und die innere Teiloberfläche 501 deutlich getrennt voneinander wahrnehmbar. Dieses Ausführungsbeispiel der Erfindung ist weiterhin in Fig. 6 nach einer Spreizung der sich herausbildenden ersten Lamelle 601 und 602 gezeigt. Dabei wird das verpackte Präservativ 600 beispielsweise durch die Elastizität des Materials in dem flexiblen Bandabschnitt gehalten, da dort Rückstellkräfte zum Beispiel in den Lamellen wirken. Dabei können diese Kräfte besonders in den Übergangsbereichen 605 zwischen den Lamellen und dem restlichen flexiblen Bandabschnitt wirken. Natürlich kann das in Figur 6 gezeigt Band vollständig aus elastischem Material gefertigt sein. Aufgrund der beiden Durchtrennungen entlang der Längsrichtung des Präservativhaltebandes kann das verpackte Präservativ durch beide Durchtrennungen durchgeführt werden Ein anschließende Fixierung ist wie gezeigt möglich. Dabei wird das verpackte Präservativ 600 von den Lamellen insbesondere von den Oberflächen der Lamellen 601, 602 und 603 umschlossen. Beispielsweise können elastische Rückstellkräfte innerhalb der Lamellen 601, 602, 603 die Fixierung verursachen. Dabei werden die Rückstellkräfte aufgrund der Spreizung bei einer Auseinanderbewegung der verschiedenen Lamellen bei der Durchführung des verpackten Präservativs erzeugt.

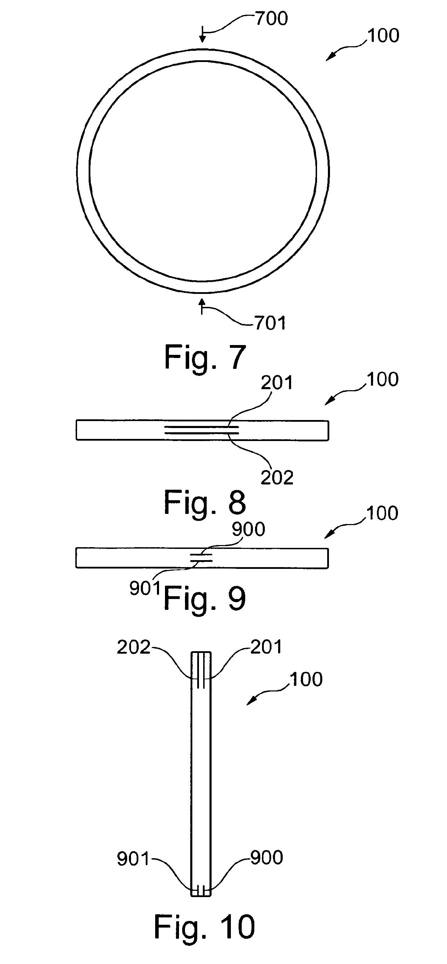

Die Fig. 7 bis 12 zeigen verschiedene Ansichten eines weiteren Ausführungsbeispiels des Präservativhaltebandes 100. Dabei ist in Fig. 7 eine Frontansicht des Präservativhaltebandes 100 gezeigt. Fig. 8 zeigt das Präservativhalteband der Fig. 7 aus der Blickrichtung 700 und Fig. 9 hingegen zeigt eine Ansicht des Präservativhaltebandes aus der Blickrichtung 701, aus der zwei weitere Durchtrennungen 900 und 901 an der Unterseite des Bandes zu erkennen sind. Die insgesamt vier Durchtrennungen 201, 202, 900 und 901 sind in der Seitenansicht der Fig. 10 deutlich zu sehen.

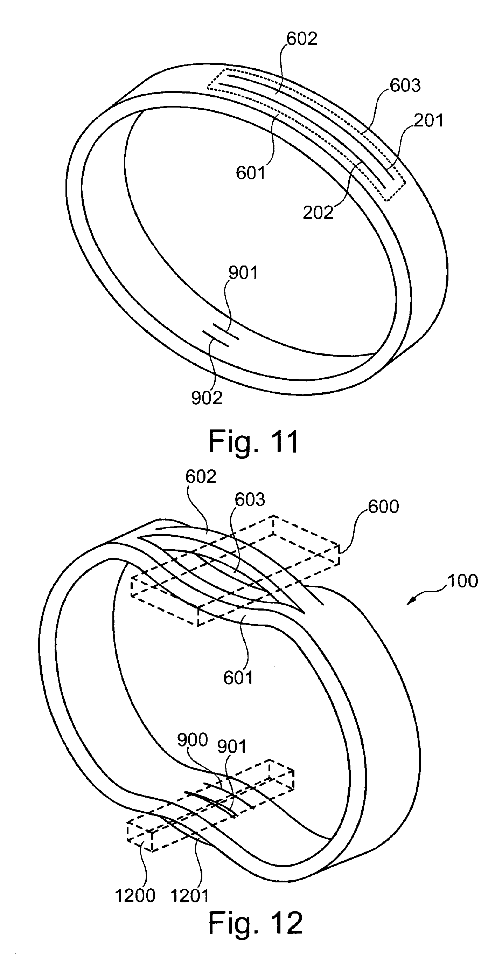

Die Fig. 11 und 12 zeigen eine perspektivische dreidimensionale Darstellung dieses Ausführungsbeispiels des Präservativhaltebandes 100. Dabei zeigt die Fig. 11 einen Grundzustand, in der die Lamellen oder Stege 601, 602 und 603, welche sich aufgrund der Durchtrennungen in dem Halteband ergeben, in einem entspannten Zustand vorliegen. Im Gegensatz dazu zeigt Fig. 12 die drei Lamellen 601, 602 und 603 in aufgespreiztem Zustand sind. Ein verpacktes Präservativ 600 ist in einem Zwischenbereich 604 zwischen den Lamellen hindurchgeführt und wird dort durch die Lamellen fixiert. Dabei liegt das verpackte Präservativ auf der ersten und der dritten Lamelle 601 und 603 auf und wird von oben durch die zweite Lamelle 602 beispielsweise mittels elastischer Rückstellkräfte gehalten. Auch eine andere Anordnung ist möglich. Ebenso ist es möglich, dass an den weiteren Durchtrennungen 900 und 901 ein weiteres verpacktes Präservativ oder auch ein Einwegprodukt, wie weiter oben beschrieben, durchgeführt ist und gehalten wird. Dabei können sich auch in diesem gezeigten unteren Bereich, in dem die weiteren Durchtrennungen liegen, weitere Lamellen oder Stege ausbilden. Eine weitere Lamelle ist mit 1201 gezeigt.

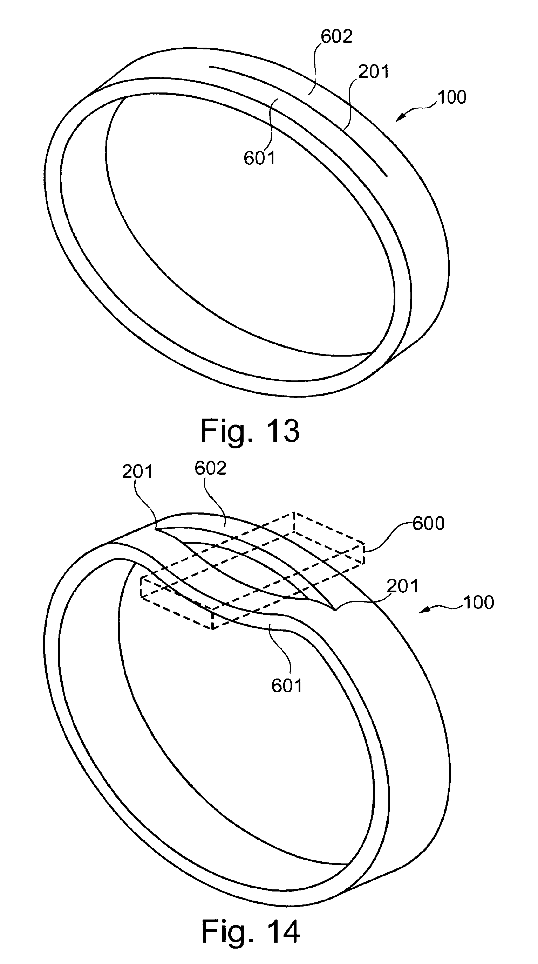

Die perspektivischen dreidimensionalen Darstellungen in Fig. 13 und 14 zeigen ein Präservativhalteband 100 gemäß einem weiteren Ausführungsbeispiel der Erfindung. Dabei ist das Präservativhalteband lediglich mit einer Durchtrennung 201 versehen.. Diese Stege oder Lamellen 601 und 602 sind in Fig. 13 in entspanntem Zustand gezeigt. In Fig. 14 hingegen sind die Lamellen nach einer Durchführung eines verpackten Präservativs 600 durch die erste Durchtrennung 201 gezeigt. Dabei ist deutlich zu sehen, dass das zu fixierende Präservativ 600 aufgrund der Umschließung des Präservativs durch die Lamellen in seiner Position gehalten wird.

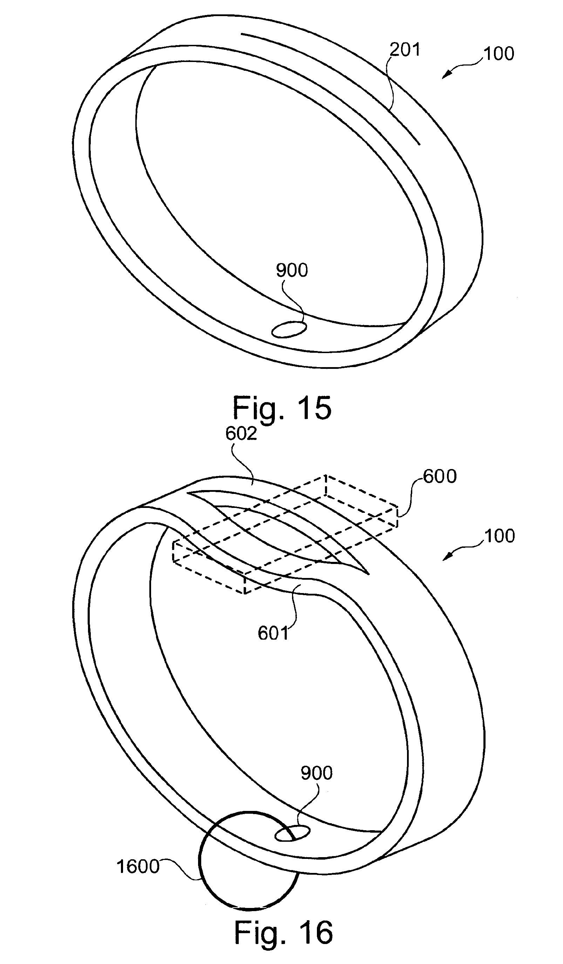

Die Fig. 15 und 16 sind perspektivische Darstellungen eines weiteren Ausführungsbeispiels des Präservativhaltebandes. Dabei weist das Präservativhalteband 100 eine weitere kreisrunde Durchtrennung 900 auf, welche weitere Durchtrennung in Längsrichtung von der ersten Durchtrennung beabstandet angeordnet ist. Dabei ist hier eine ringförmig geschlossene Form des Haltebandes gezeigt. In Fig. 16 ist weiterhin das Präservativhalteband 100 im bestückten Zustand und mit einem Schlüsselring 1600 gezeigt. Dabei kann der Schlüsselring 1600 an dem Präservativhalteband durch die weitere Durchtrennung 900 fixiert werden.

Ergänzend ist darauf hinzuweisen, dass Bezugszeichen in den Ansprüchen nicht als Einschränkung anzusehen sind.

### LISTE DER BEZUGSZEICHEN

- 100: Präservativhalteband
- 101: Kontur der äußeren Teiloberfläche
- 102: Kontur der inneren Teiloberfläche
- 103: Blickrichtung
- 104: Blickrichtung
- 200: flexibler Bandabschnitt
- 201: erste Durchtrennung
- 202: zweite Durchtrennung
- 203: Längsrichtung
- 204: Länge des flexiblen Bandabschnitts
- 205: Breite des flexiblen Bandabschnitts
- 500: äußere Teiloberfläche
- 501: innere Teiloberfläche
- 600: verpacktes Präservativ
- 601: erste Lamelle
- 602: zweite Lamelle
- 603: dritte Lamelle
- 604: Zwischenbereich
- 605: Übergang zwischen Lamelle und restlichem flexiblen Bandabschnitt
- 700: Blickrichtung
- 701: Blickrichtung
- 900: weitere Durchtrennung
- 901: weitere Durchtrennung
- 1200: weiteres verpacktes Präservativ/Einwegprodukt
- 1201: weitere Lamelle
- 1600: Schlüssehring/Gegenstand befestigt an weiterer Durchtrennung

## Patentansprüche

1. Präservativhalteband für die Aufbewahrung und den Transport eines Präservativs an einem Handgelenk, das Präservativhalteband aufweisend:
einen flexiblen Bandabschnitt (200),
wobei der flexible Bandabschnitt (200) entlang einer Längsrichtung (203) eine erste Durchtrennung (201) aufweist;
wobei die erste Durchtrennung (201) derart ausgeführt ist, dass ein verpacktes Präservativ durch die erste Durchtrennung durchführbar ist;
wobei die erste Durchtrennung (201) derart ausgeführt ist, dass das verpackte Präservativ nach einer Durchführung in dem flexiblen Bandabschnitt (200) fixierbar ist;
wobei das Präservativhalteband aus einem elastischen Material besteht; und
wobei das Präservativhalteband einstückig ausgeführt ist.

2. Präservativhalteband gemäß Anspruch 1, das Präservativhalteband weiterhin aufweisend:
eine zweite Durchtrennung (202) in dem flexiblen Bandabschnitt (200);
wobei der flexible Bandabschnitt (200) eine Breite und eine Länge aufweist;
wobei die erste und zweite Durchtrennung (201, 202) in der Breite nebeneinander angeordnet sind und in Längsrichtung wenigstens teilweise auf gleicher Höhe angeordnet sind;
wobei die erste und zweite Durchtrennung (201, 202) derart ausgeführt sind, dass ein verpacktes Präservativ sowohl durch die erste, als auch durch die zweite Durchtrennung durchführbar ist; und
wobei die erste und zweite Durchtrennung (201, 202) derart ausgeführt sind, dass das verpackte Präservativ nach einer Durchführung in dem flexiblen Bandabschnitt fixierbar ist.

3. Präservativhalteband gemäß einem der vorherigen Ansprüche,
wobei das Präservativhalteband aus einem Material besteht, welches ausgewählt ist aus der Gruppe bestehend aus Kautschuk, Latex, Gummiharz, andere verarbeitete kautschukähnliche Pflanzensäfte, vulkanisiertes Material mit und ohne synthetischen Kautschukanteilen, und Material, das beim Eintrocknen durch Polymerisation zu einem elastischen Feststoff verhärtet ist.

4. Präservativhalteband gemäß einem der Ansprüche 2 bis 3,
wobei die erste und die zweite Durchtrennung (201, 202) im Wesentlichen parallel entlang der Längsrichtung (203) des Präservativhaltebandes verlaufen.

5. Präservativhalteband gemäß einem der Ansprüche 2 bis 4,
wobei die erste und die zweite Durchtrennung (201, 202) im Wesentlichen die gleiche Länge aufweisen.

6. Präservativhalteband gemäß einem der vorherigen Ansprüche,
wobei das Präservativhalteband ringförmig geschlossen ausgeführt ist.

7. Präservativhalteband gemäß einem der vorherigen Ansprüche, das Präservativhalteband weiterhin aufweisend:
eine weitere Durchtrennung (901) an einer von der ersten Durchtrennung (201) in Längsrichtung beabstandeten Stelle des Präservativhaltebandes.

8. Präservativhalteband gemäß Anspruch 7,
wobei die weitere Durchtrennung (901) zur Aufnahme eines Rings oder eines Schlüssels ausgeführt ist.

9. Präservativhalteband gemäß einem der vorherigen Ansprüche,
wobei sich auf Grund der ersten Durchtrennung (201) zumindest zwei Lamellen an einer Oberfläche des flexiblen Bandabschnitts (200) ausbilden;
wobei die beiden Lamellen zur Durchführung des verpackten Präservativs ausgeführt sind; und
wobei mittels der Durchführung eine Fixierung des verpackten Präservativs auf Grund einer Flexibilität der beiden Lamellen erfolgt.

10. Verwendung eines Präservativhaltebandes gemäß einem der Ansprüche 1 bis 9 zur Aufbewahrung und zum Transport eines Präservativs.

## Claims

1. A condom retaining strap for the safekeeping and transport of a condom on a wrist, the condom retaining strap having:
a flexible strap section (200); wherein
the flexible strap section (200) has a first cut (201) along a lengthwise direction (203), wherein
the first cut (201) is embodied such that a packaged condom can be passed through the first cut, wherein
the first cut (201) is embodied such that after being passed through a packaged condom can be fixed in the flexible strap section (200), wherein
the condom retaining strap consists of an elastic material, and wherein
the condom retaining strap is embodied in one piece.

2. The condom retaining strap in accordance with Claim 1, the condom retaining strap furthermore having:
a second cut (202) in the flexible strap section (200); wherein
the flexible strap section (200) has a width and a length; wherein
the first and second cuts (201, 202) are arranged adjacent to one another across the width, and in the lengthwise direction are arranged at least partly at the same height; wherein
the first and second cuts (201, 202) are embodied such that a packaged condom can be passed through both the first and second cuts, and wherein
the first and second cuts (201, 202) are embodied such that after being passed through the packaged condom can be fixed in the flexible strap section.

3. The condom retaining strap in accordance with one of the preceding claims, wherein
the condom retaining strap consists of a material, which is selected from the group consisting of natural rubber, latex, gum resin, other processed plant juices similar to natural rubber, vulcanised material with and without synthetic rubber components, and material that when dried out by polymerisation is hardened to an elastic solid.

4. The condom retaining strap in accordance with one of the Claims 2 to 3, wherein
the first and second cuts (201, 202) run essentially parallel along the lengthwise direction (203) of the condom retaining strap.

5. The condom retaining strap in accordance with one of the Claims 2 to 4, wherein
the first sand second cuts (201, 202) have essentially the same length.

6. The condom retaining strap in accordance with one of the preceding claims, wherein
the condom retaining strap is embodied as a closed annular shape.

7. The condom retaining strap in accordance with one of the preceding claims, the condom retaining strap
furthermore having:
a further cut (901) at a location of the condom retaining strap spaced apart from the first cut (201) in the lengthwise direction.

8. The condom retaining strap in accordance with Claim 7, wherein
the further cut (901) is embodied for the purpose of accommodating a ring or a key.

9. The condom retaining strap in accordance with one of the preceding claims, wherein
based in the first cut (201) at least two lamellae are developed on a surface of the flexible strap section (200); wherein
the two lamellae are embodied for purposes of passing through the packaged condom; and wherein
by means of the passing through a fixing of the packaged condom takes place based on a flexibility of the two lamellae.

10. The use of a condom retaining strap in accordance with one of the Claims 1 to 9 for the safekeeping and transport of a condom.

## Revendications

1. Bande de retenue de préservatif pour la conservation et le transport d'un préservatif à un poignet, la bande de retenue de préservatif comportant :
une portion de bande souple (200),
dans laquelle la portion de bande souple (200) comporte une première découpe (201) le long d'une direction longitudinale (203),
dans laquelle la première découpe (201) est réalisée de telle sorte qu'un préservatif emballé peut passer à travers la première découpe,
dans laquelle la première découpe (201) est réalisée de telle sorte que le préservatif emballé peut être fixé après un passage dans la portion de bande souple (200),
dans laquelle la bande de retenue de préservatif est constituée d'un matériau élastique, et
dans laquelle la bande de retenue de préservatif est réalisée d'un seul tenant.

2. Bande de retenue de préservatif selon la revendication 1, la bande de retenue de préservatif comportant en outre :
une seconde découpe (202) dans la portion de bande souple (200),
dans laquelle la portion de bande souple (200) a une largeur et une longueur,
dans laquelle les première et seconde découpes (201, 202) sont disposées côte à côte dans la largeur et sont disposées au moins partiellement à la même hauteur dans la direction longitudinale,
dans laquelle les première et seconde découpes (201, 202) sont réalisées de telle sorte qu'un préservatif emballé peut passer aussi bien à travers la première découpe qu'à travers la seconde découpe, et
dans laquelle les première et seconde découpes (201, 202) sont réalisées de telle sorte que le préservatif emballé peut être fixé après un passage dans la portion de bande souple.

3. Bande de retenue de préservatif selon l'une quelconque des revendications précédentes,
dans laquelle la bande de retenue de préservatif est constituée d'un matériau qui est choisi parmi le groupe constitué de caoutchouc, de latex, de caoutchouc-résine, d'autres sèves traitées analogues au caoutchouc, de matériau vulcanisé avec et sans fractions de caoutchouc synthétique, et d'un matériau qui durcit en séchant pour former un matériau solide élastique par polymérisation.

4. Bande de retenue de préservatif selon l'une quelconque des revendications 2 à 3,
dans laquelle les première et seconde découpes (201, 202) s'étendent de manière sensiblement parallèle le long de la direction longitudinale (203) de la bande de retenue de préservatif.

5. Bande de retenue de préservatif selon l'une quelconque des revendications 2 à 4, dans laquelle les première et seconde découpes (201, 202) ont sensiblement la même longueur.

6. Bande de retenue de préservatif selon l'une quelconque des revendications précédentes,
dans laquelle la bande de retenue de préservatif est réalisée sous forme d'anneau fermé.

7. Bande de retenue de préservatif selon l'une quelconque des revendications précédentes, la bande de retenue de préservatif comportant en outre :
une découpe supplémentaire (901) à un endroit de la bande de retenue de préservatif espacé de la première découpe (201) dans la direction longitudinale.

8. Bande de retenue de préservatif selon la revendication 7,
dans laquelle la découpe supplémentaire (901) est réalisée de manière à recevoir un anneau ou une clé.

9. Bande de retenue de préservatif selon l'une quelconque des revendications précédentes,
dans laquelle au moins deux lamelles se forment sur une surface de la portion de bande souple (200) en raison de la première découpe (201),
dans laquelle les deux lamelles sont réalisées de manière à faire passer le préservatif emballé, et
dans laquelle le passage permet une fixation du préservatif emballé en raison de la souplesse des deux lamelles.

10. Utilisation d'une bande de retenue de préservatif selon l'une quelconque des revendications 1 à 9 pour la conservation et le transport d'un préservatif.
